# EUROPEAN PATENT APPLICATION

(11) **EP 4 449 986 A1**
(43) Date of publication of application: **23.10.2024**
(21) Application number: 23169083.5
(22) Date of filing: 20.04.2023
(51) Int. Cl.: A61B 5/103, A61B 5/00, D04B 1/00

(54) **FOOTWEAR FOR MONITORING A WEARER`S DIABETIC FOOT**

(30) Priority: 19.04.2023 PT 2023118609
(71) Applicant: Escola Superior de Enfermagem de Coimbra, 3004-011 Coimbra (PT); ICC - Industrias E Comércio de Calçado, SA, 4810-718 Guimaraes (PT); Universidade do Minho, 4710-057 Braga (PT); Universidade de Coimbra, 3004-531 Coimbra (PT)
(72) Inventor: DE SOUSA SALGUEIRO OLIVEIRA, ANABELA, 3030-175 COIMBRA (PT); Alves Apóstolo, João Luis, 3045-120 COIMBRA (PT); Baptista Sousa, Liliana, 3140-305 Pereira, MMV (PT); dos Santos Dinis Parreira, Pedro Miguel, 3045-293 Ameal - Coimbra (PT); Franco de Almeida, Inês, 3840-344 Lavandeira (PT); Negrão Baptista, Rui Carlos, 3045-043 Coimbra (PT); Ribeiro Gonçalves Leite, Teófilo Óscar, 4810-718 Guimarães (PT); Contente Rangel Henriques, Mariana, 4710-057 Braga (PT); Soares de Carvalho, Isabel, 4710-057 Braga (PT); de Almeida Whiteman Catarino, André Paulo, 4150-143 Porto (PT); Fernandes Carvalho, Sandra Maria, 4705-289 Braga (PT); Moura Duarte, Fernando, 4700-416 Braga (PT); Alves Bernardes, Rafael, 3000-187 Coimbra (PT); Machado Viana, Júlio César, 4705-204 Braga (PT); Ribeiro dos Santos, Ana Raquel, 4835-065 Guimarães (PT); Moreira Ferreira da Rocha, Ana Maria, 4800-019 Guimarães (PT); Pacheco Oliveira, Natálie, 4710-057 Braga (PT)
(74) Representative: Patentree

(57) **Abstract**

Footwear for monitoring a wearer's diabetic foot with a sensorized insole and a data processor configured for measuring pressure, humidity and temperature in a plurality of predetermined zones of the foot, wherein the sensorized insole comprises sensors coupled to said zones for detecting pressure, humidity and temperature of the foot; wherein the insole comprises a knitted fabric having a mesh structure with a plurality of rows and columns wherein the columns comprise loops of weft mesh to increase the volume of the mesh structure.

## Description

### TECHNICAL FIELD

The present disclosure relates to a footwear for monitoring a wearer's diabetic foot with a sensorized insole and a data processor configured for measuring pressure, humidity and temperature in a plurality of predetermined zones of the foot.

### BACKGROUND

Document WO2009090509 discloses a portable and autonomous device and corresponding method for the measurement, recording and calculation of dynamic parameters of the pedestrian locomotion of its bearer. The method hereby disclosed allows determining the distance effectively traveled, the velocity of displacement and the pressures exerted on the contact surface of the lower limb with the ground, during the pedestrian locomotion, according to its different phases. Through sensors strategically placed, accelerations, angular velocities and pressures are measured and recorded on the control unit. The device disclosed includes a processing unit that implements the method disclosed and a communication unit to transfer to an external unit the calculated parameters. The disclosed invention can be used in applications that involve monitoring parameters of the locomotion activity or daily ambulation of its bearer, in the areas of health and sports or in any physical and occupational activities.

Document PT103551A discloses a sensing system for the monitoring of posture, orientation and movement of a body in a three-dimensional space, differentiating among the various stationary states and transient states in what the user can be. Sensing modules are composed by three-axis accelerometers, three-axis magnetometers and interfacing electronics. The device is encapsulated, enabling its use in adverse and harsh environments. The data monitored by the sensing modules are transmitted in real time, by the local communication device, to the central communication device, using a 2.4 GHz RF transceiver. Generic applications derived from the analysis of the monitored data include the rectification of a user's incorrect body, thereby avoiding injuries, the acceleration of the healing process in therapy, or the simple monitoring of a user's physical activity. Being compact and encapsulated, this device can be used to monitor and analyze the movement of athletes during physical activity, such as a swimmer.

These facts are disclosed in order to illustrate the technical problem addressed by this disclosure.

### GENERAL DESCRIPTION

The present disclosure relates to a footwear for monitoring a wearer's diabetic foot.

This disclosure aims to respond to a market need in what concerns the creation of a fully adjusted and adapted footwear for the clinical condition of the diabetic foot. This disclosure proposes the development of a new type of footwear oriented to the therapeutic footwear segments, targeting the clinical condition of the Diabetic Foot (DF). Currently, the risk of developing this clinical condition is estimated to be between 19-34% of people diagnosed with Diabetes. In addition to the severity and complications associated with DP, this clinical condition also results in a huge financial burden.

Disclosed is a footwear for monitoring a wearer's diabetic foot with a sensorized insole and a data processor configured for measuring pressure, humidity and temperature in a plurality of predetermined zones of the foot, wherein the sensorized insole comprises sensors coupled to said zones for detecting pressure, humidity and temperature of the foot; wherein the insole comprises a knitted fabric having a mesh structure with a plurality of rows and columns wherein the columns comprise loops of weft mesh to increase the volume of the mesh structure.

In an embodiment, said mesh structure has a stitch structural cell (i.e pattern repeat) wherein columns have floating loops combined with normal and/or loaded loops.

In an embodiment, said mesh structure has a stitch structural cell wherein columns of floating loops alternate with columns of normal and/or loaded loops.

In an embodiment, said mesh structure has a stitch structural cell comprising a loop alternation zone wherein columns of floating loops alternate with columns of normal and/or loaded loops.
In an embodiment, said mesh structure has a stitch structural cell comprising four loop alternation zones arranged in a grid pattern.

In an embodiment, said mesh structure has a stitch structural cell comprising two loop alternation zones arranged diagonally in a grid pattern of four zones.

In an embodiment, the sensorized insole is a flexible polymeric substrate, in particular in a polymeric film (e.g., PET, PC, PA, PI), with printed conductive tracks and pressure, temperature and humidity sensors.

In an embodiment, a cleaning insole according to the type of curvature of the plantar arch is placed over the sensorized insole, and these can be easily separated or not.

In an embodiment, the sensors are in active thin films or traces, glued, deposited or printed over the conductive tracks printed on the sensorized insole.

In an embodiment, the sensors are in active thin films or traces, in particular, in flexible material sensitive to pressure (e.g., piezoresistive polymers or polymeric composites), to temperature (e.g., thermoresistive, polymeric or metallic materials), to humidity (e.g., humidity-sensitive polymers or polymeric composites).

In an embodiment, pressure, temperature and humidity sensors can be commercially available, and assembled or glued to the sensorized insole.

In an embodiment, a cleaning insole according to the type of curvature of the plantar arch is placed over the sensorized insole, and these can be easily separated or not.

In an embodiment, the sensors are in active thin films or traces, glued, deposited or printed over the conductive tracks printed on the sensorized insole.

In an embodiment, the sensors are in active thin films or traces, in particular in flexible material sensitive to pressure, to temperature and to humidity.

In an embodiment, the cleaning insole comprises a silver oxide or titanium nitrite doped with silver coating for antimicrobial application.

In an embodiment, the coating thickness is less than 2 µm, preferably less than 1.5 µm.

In an embodiment, the composition is administered coated in a substrate of a shoe or coated in a substrate of a part of the footwear.

In an embodiment, the rows are knitted with two types of yarns, in particular a first yarn to manage temperature and humidity and a second yarn to promote mesh elasticity and increased mesh thickness.

In an embodiment, the first yarn has a linear mass ranging from 15 - 25 tex; and the second yarn has an elastane monofilament with a linear mass of 2-4 tex.

In an embodiment, the first yarn is composed of 70% (w/w) cotton and 30% (w/w) viscose, preferably with Outlast^{®} or the first yarn is composed of 85% (w/w) polyester, preferably with Dri Release^{®} technology, and 15% (w/w) cotton; and the second yarn has an elastane monofilament.

In an embodiment, the areas of greatest pressure are identified by the data processor to promote the prevention and treatment of the user's diabetic foot.

In an embodiment, the areas of greatest pressure, temperature and humidity are identified by the data processor to promote the prevention and treatment of the user's diabetic foot.

In an embodiment, the knitted fabric has a mesh structure with a thickness between 5 mm to 10.8 mm, preferably between 9 mm to 9.8 mm, more preferably 6 mm.

In an embodiment, such footwear is a shoe, boot, slipper, or sneaker.

### BRIEF DESCRIPTION OF THE DRAWINGS

The following figures provide preferred embodiments in order to illustrate the disclosure and should not be considered as limiting the scope of the invention.
**Figure 1** represents a schematic representation of an embodiment of the system's functional diagram.
**Figure 2** represents an embodiment of the sensorized insole with the identification of the plantar pressure points.
**Figure 3** represents a stitch structural cell (CEP - *Célula Estrutural do Ponto)* of the meshes with 3D effect.
**Figure 4** represents an aspect of the technical back of the meshes with 3D effect.

### DETAILED DESCRIPTION

The present disclosure refers to a footwear for monitoring a wearer's diabetic foot with a sensorized insole and a data processor configured for measuring pressure, humidity and temperature in a plurality of predetermined zones of the foot.

The present disclosure integrates footwear components and a removable insole, adding new materials at the insole and sole level that allow (i) greater shock absorption; (ii) shape adaptability, (iii) greater recovery after loading and less abrasion; and (iv) the ability to reduce the risk of pressure ulcers.

The insoles are functionalized with antimicrobial coatings in order to prevent the occurrence of infections resulting from DF.

The incorporation of materials at the footwear's interior level aims for greater ergonomic and thermo-physiological comfort, and the integration of form-fitting materials aims to promote superior performance.

In terms of design, it has decorative coatings for the outer part of the footwear, providing an appealing design for end users, integrating self-sensing materials for pressure, temperature and humidity monitoring, allowing the detection of plantar pressure zones and other critical areas of the foot.

The inclusive system for reading critical plantar pressure, temperature and humidity points throughout continuous footwear use consists of a sensorized insole (flexible film), a cleaning insole and a data acquisition and storage electronic system, powered by a rechargeable battery. The sensorized insole is connected to the data acquisition system via a connector. The sensors are placed in critical areas of the foot sole, previously identified as areas of higher pressure, humidity and temperature, and susceptible to greater load and friction on the foot. Their position is also adjusted according to the footwear size. The electronic system and the battery are placed under the foot, in areas of lower pressure and less flexibility (e.g., under the arch of the foot). The system aims to identify areas of higher pressure, humidity and temperature, and maintained throughout the continued use of the footwear, and that can lead to possible diabetic foot ulcers. Once the system identifies high maintained values, the healthcare professional analyzing the data will recommend measures for the mitigation of those values, which may include the use of a more appropriate mounting/cleaning insole. The proposed solution of a sensorized insole allows the use of different mounting/cleaning insoles, suitable for the user.

The sensorized insole developed was based on electronics printed on a flexible polymeric substrate and the sensors on active films. The polymeric substrate is printed with conductive ink (e.g., Ag, C, etc.) and the electrically conductive tracks that connect the sensors to a connector are produced. The insole incorporates three types of sensors, all resistive: pressure, temperature and humidity. As they all are resistive sensors, it simplifies the interfacing electronics of the data acquisition system. The sensors are thin and placed on the polymeric substrate, with the following characteristics:
The pressure sensor is based on interdigital electrodes on which a piezoresistive active film is placed. Typically, this film is based on an elastomer charged with conductive particles (CNT, Ag, C, etc.), such as Velostat^{®}, or another polymer. The electrical resistivity of these materials decreases with pressure, due to their deformation. This sensor is coated with a thin protective material for insulation and increased durability.
The humidity sensor is based on interdigital electrodes on which a humidity-sensitive active film is placed. Typically, this film is based on a hygroscopic polymer (e.g., PVA, PU) charged with conductive particles. The electrical resistivity of these materials increases with humidity, due to the expansion of the polymer causing the conductive charges to move away. This sensor is protected by a porous membrane that allows humidity, but not liquids, to pass through.
The temperature sensor is based on electrodes connected by a thermo-resistive material, i.e. a material in which the electrical resistivity varies with temperature. For example, a conductive ink that can be printed between the two electrodes, based on Ni, Pt, C, or a conductive polymer (e.g., PEDOT:PSS). This sensor is coated with a thin protective material for insulation and increased durability.

The insole has a connector to connect to the interfacing electronics that allows reading, operating and storing the data read from the sensors. The connector can be a commercial one of various types and can be mounted in various ways, such as mounted on the insole by SMD (surface mounted devices) technology, connected to the insole using a FFC (flexible flat cable) or FPC (flexible printed circuit) connector, or similar. The latter has the advantage of increasing the flexibility of the connection between the sensorized insole and the interfacing electronics, extending the lifetime of the connection.

Interfacing electronics may comprise data acquisition system that reads sensor signals, performs operations on the data, saves and stores them in a memory internal to the circuit (e.g., RAM) or external (e.g., micro-SD disk). In another configuration, the data read or processed can be sent wirelessly to a receiving device, where further operations on the data can be performed. The data acquisition and recording rate is defined according to the application and dimensions of the electronic systems. It should be as small as possible, so that it is hidden in the shoe. External solutions, which may stigmatize the use of the developed solution, are not allowed.

The electronic system is powered by a rechargeable battery.

The electronic system and the battery are placed in the sole of the shoe, under the sensorized insole. The entire assembly is removable from the shoe, allowing its removal, replacement, and battery charging. A mounting and cleaning insole is placed over the sensorized insole, also removable, which allows for a better fit to the foot type, a better distribution of plantar pressures, and for cleaning thereof. The sensorized insole is not in direct contact with the foot.

The sensorized insole described above can be manufactured with commercial sensors:
Pressure sensors of the FSR (Force Sensing Resistors) type, however, the connections of the sensors to the conductive tracks are fragile zones, decreasing the durability of the solution
Small resistive humidity sensors are not available; electrical impedance sensors, or other, would have to be selected, which makes the interfacing electronics more complex and larger. In addition, these commercial sensors are rigid.
Temperature sensors of the type, thermocouple, thermistor, IC, however these sensors are rigid and low deformable, and can cause pressure points on the foot, in addition to their poor durability in the case of high deformation.

Lining component and insole covering: A weft or warp mesh is usually characterized by a textile substrate, which is manufactured on a weft or warp knitting machine and whose spatial geometry reveals a thickness significantly smaller than its width and length. A weft or warp mesh is considered to have a 3D effect if its thickness significantly exceeds the normal thickness of a so-called simple mesh. The 3D effect is achieved in several ways, namely by the stitch structural cell (CEP), the raw material, and also by the characteristics of the knitting machine on which it is manufactured. The meshes with 3D effect herein described were developed on a knitting machine with a needle system, with certain raw materials and specific CEP designs, capable of projecting the third spatial dimension - thickness - to values between 2 and 6 times the thickness of a plain mesh.

According to the requirements of the product in question, meshes are produced with raw materials that allow, on the one hand, a more efficient management of temperature and humidity, an ability to increase the thickness, providing increased impact absorption and also a greater ability to recover from deformation. Thus, the rows were knitted with two types of textile yarn simultaneously: Yarn (A), responsible for managing temperature and humidity; Yarn (B), responsible for contributing to the elasticity of the mesh and increasing the thickness of the mesh. For the purposes of developing the prototype, as yarn (A) was used a 40 Ne linear mass yarn composed of 70% cotton and 30% viscose with Outlast^{®}, or alternatively a 30 Ne linear mass yarn (A) composed of 85% polyester with Dri Release^{®} technology and 15% cotton. As yarn (B) an elastane monofilament with linear mass 33 Nm was used. Any textile yarn that meets the requirements defined for the shoe can be used as an alternative to those proposed. The linear masses of the yarns will depend on the set of the knitting machine used, and for the prototype a 24 E set knitting machine was used.

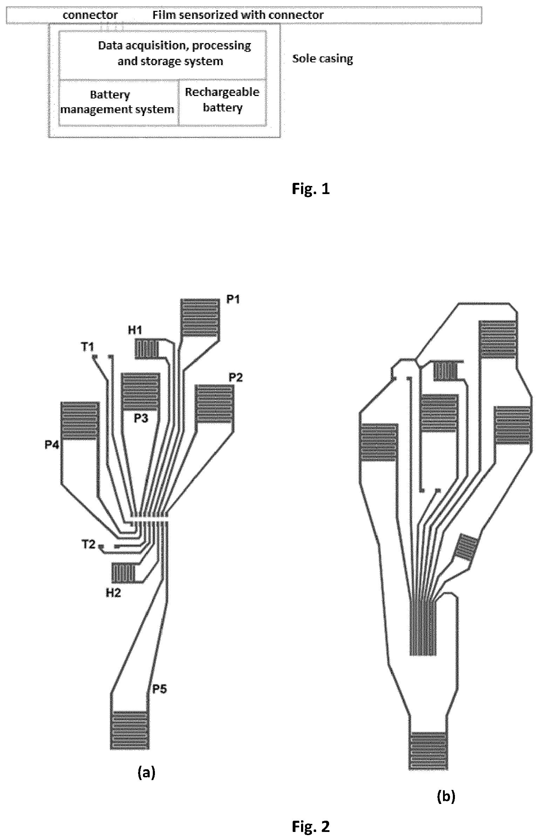

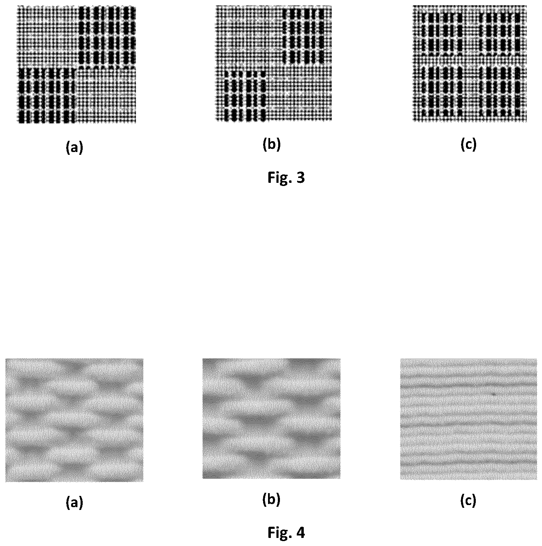

The mesh structures used in the prototype consist of a combination of loops of weft mesh, namely normal or loaded loops and floating loops. The combination according to appropriate patterns in the CEP, allow the projection of the third dimension, the thickness, to significantly larger dimensions, than those achieved with the CEP used in simple weft meshes. This projection with resulting increased thickness is seen on the technical back of the mesh. The combination of floating loops with normal and/or loaded loops is done in the form of alternating columns of floating loops with columns of normal and/or loaded loops, as can be seen in the examples of **Figure 3****,** examples based on normal and floating loops. The increased length of the floating loop columns, as well as the selected pattern, contribute to the increased thickness of the mesh.
The CEP used for the development of the proposed weft mesh structures for the inside of the shoe are as illustrated in **Figure 1****,** in which the normal loops are represented in lighter/softer, and the floating loops are represented in darker/deeper.

**Figure 3** shows a schematic representation of an embodiment of a weft mesh structure whose CEP consists of 32 rows and 32 columns. The black columns result in a loop retention effect, which leads to the creation of small cells, resulting in an increase in mesh thickness, as shown in **Figure 4****.a.** **Figure 3****.b** shows a weft mesh structure whose CEP consists of 32 rows and 32 columns with the difference to the previous one of having more columns knitted with normal loops and fewer columns of floating loops, resulting in a distinct projection effect **(Figure 4.b).** **Figure 3****.c** shows a weft mesh structure whose CEP consists of 32 rows and 32 columns, in which the columns with floating loops are interrupted by normal loops. This construction results in a higher density of retained loops, but by having shorter floating loop columns results in a mesh with a smaller thickness and continuous appearance **(Figure 4.c).**

The produced meshes were compared with other meshes, namely jersey and spacer meshes. The thicknesses recorded for these meshes ranged between 3.0 mm and 3.6 mm, an increase of 2 to 3 times greater than that achieved with a jersey mesh produced under the same conditions. Higher thicknesses can be achieved by changing the CEP and can reach more than 6 mm. It has also been found that the meshes produced perform identically or better in terms of thermal behavior when compared to plain meshes such as jersey, produced with the same raw materials and conditions. It was also found that these 3D effect meshes are able to withstand a much higher compressive strain than jersey, and also show resilience. The 3D effect that is created by the combination of CEP and raw material can also contribute to the promotion of micro blood circulation.

The 3D meshes herein described can be applied to the lining of the shoe, on the inside of the vamp, and can completely or partially cover that lining and/or the interior, so as to cover the inside of the shoe. They can also be applied on the surface of the insole that is in direct contact with the user's foot.

In an embodiment, the weft mesh produced from a CEP can be obtained by combining columns of floating loops with columns of normal and/or loaded loops.

In an embodiment, the weft mesh can be produced using two textile or other yarns, technically able to be knitted simultaneously.

In an embodiment, the weft mesh can be produced so that one of the yarns is either bare or coated elastane monofilament, with the other yarn being of material suitable for the intended purpose.

The term "comprising" whenever used herein is intended to indicate the presence of features, integers, steps, components mentioned, but not to preclude the presence or addition of one or more other features, integers, steps, components or groups thereof.

It will be appreciated by a person with ordinary skill in the art that, unless otherwise indicated herein, the particular sequence of steps described is illustrative only and may be varied without departing from the disclosure. Thus, unless otherwise indicated, the steps described are thus disordered, meaning that, when possible, the steps may be performed in any convenient or desirable order.

The disclosure should not be seen in any way restricted to the embodiments described, and a person with ordinary skill in the art may foresee many possibilities for modifications thereof. The above described embodiments are combinable. The following claims further define particular embodiments of the disclosure.

## Claims

1. Footwear for monitoring a wearer's diabetic foot with a sensorized insole and a data processor configured for measuring pressure, humidity and temperature in a plurality of predetermined zones of the foot,
wherein the sensorized insole comprises sensors coupled to said zones for detecting pressure, humidity and temperature of the foot;
wherein the insole comprises a knitted fabric having a mesh structure with a plurality of rows and columns wherein the columns comprise loops of weft mesh to increase the volume of the mesh structure.

2. Footwear according to the preceding claim wherein said mesh structure has a stitch structural cell wherein columns have floating loops combined with normal and/or loaded loops.

3. Footwear according to any one of the preceding claims wherein said mesh structure has a stitch structural cell wherein columns of floating loops alternate with columns of normal and/or loaded loops.

4. Footwear according to any one of the preceding claims wherein said mesh structure has a stitch structural cell comprising a loop alternation zone wherein columns of floating loops alternate with columns of normal and/or loaded loops.

5. Footwear according to any one of the preceding claims wherein said mesh structure has a stitch structural cell comprising four loop alternation zones arranged in a grid pattern.

6. Footwear according to any one of the preceding claims wherein said mesh structure has a stitch structural cell comprising two loop alternation zones arranged diagonally in a grid pattern of four zones.

7. Footwear according to any one of the preceding claims wherein the sensorized insole is a flexible polymeric substrate with printed conductive tracks and pressure, temperature and humidity sensors.

8. Footwear according to any one of the preceding claims wherein the sensors are in active thin films or traces, glued, deposited or printed over the conductive tracks printed on the sensorized insole.

9. Footwear according to any one of the preceding claims further comprising a cleaning insole comprising a silver oxide or titanium nitrite doped with silver coating for antimicrobial application.

10. Footwear according to the preceding claim wherein the coating thickness is less than 2 µm, preferably less than 1.5 µm.

11. Footwear according to any one of claims 9 - 10 wherein the composition is administered coated in a substrate of a shoe or coated in a substrate of a part of the footwear.

12. Footwear according to any one of the preceding claims wherein the rows are knitted with two types of yarns, in particular a first yarn to manage temperature and humidity and a second yarn to promote mesh elasticity and increased mesh thickness.

13. Footwear according to the preceding claim wherein the first yarn has a linear mass ranging from 15 - 25 tex; and the second yarn has an elastane monofilament with a linear mass of 2-4 tex.

14. Footwear according to any one of the preceding claims wherein the knitted fabric has a mesh structure with a thickness between 5 mm to 10.8 mm, preferably between 9 mm to 9.8 mm, more preferably 6 mm.

15. Footwear according to any one of the preceding claims wherein said footwear is a shoe, boot, slipper, or sneaker.
